# EUROPEAN PATENT APPLICATION

(11) **EP 3 925 961 A1**
(43) Date of publication of application: **22.12.2021**
(21) Application number: 20881409.5
(22) Date of filing: 16.10.2020
(51) Int. Cl.: C07F 9/54, A61K 31/662, A61P 39/00

(54) **MITOCHONDRIA-TARGETING COMPOUND AND COMPOSITION COMPRISING SAME FOR TREATING OR PREVENTING AGING-RELATED DISEASES**

(30) Priority: 30.10.2019 KR 20190136730
(71) Applicant: Fusion Biotechnology Co., Ltd., Daejeon 34051 (KR)
(72) Inventor: KIM, Chae Wook, Yongin-si Gyeonggi-do 16872 (KR)
(74) Representative: Vossius & Partner Patentanwälte Rechtsanwälte mbB
(86) International application number: PCT/KR2020/014185
(87) International publication number: WO 2021/085906

(57) **Abstract**

Provided are a mitochondria-targeting compound and a composition including the same for the treatment or prevention of an aging-related disease. A compound according to an aspect or a pharmaceutically acceptable salt thereof, and a pharmaceutical composition including the same are effectively used to prevent or treat aging-related diseases by specifically inducing apoptosis of senescent cells.

## Description

### TECHNICAL FIELD

Embodiments of the present disclosure relate to a mitochondria-targeting compound and a composition for the treatment or prevention of aging-related diseases including the same.

### BACKGROUND ART

The aging process is a phenomenon in which cells age and the biochemical or physiological functions of various bodies decrease over time. As we age, senescent cells accumulate in vivo, which affect diseases related to aging, limiting human lifespan.

Cell aging means that cells no longer divide, and known examples thereof are replication aging in which when the cell divides, due to the exhaustion of the telomere length, which is the nucleotide sequence at the end of the chromosome, the cell no longer divides; and cell aging induced by stress that is caused by a mechanism to suppress cancer occurrence due to external stimuli or stress, such as ultraviolet rays, viral infections, and chemicals. These senescent cells secrete more specific growth factors or physiologically active substances called senescent cell secretion activity (SASP) compared to normal cells, thereby affecting the microenvironment of adjacent cellular tissues or extracellular matrix, and being involved in various physiologically active reactions, such as inflammatory reactions and bio-homeostasis.

In general, senescent cells that have secretory activities related to the inflammatory reaction are removed by the immune system after performing various physiological roles to maintain biohomeostasis, so that the living organism performs healthy life activities in a stable state. However, as the age increases, the immune system degenerates and the self-healing ability of cells decreases. As a result, senescent cells gradually accumulate in the body, and eventually the organism lives with chronic inflammation in an abnormal state, increasing the likelihood of getting cancer or various senile diseases.

Therefore, if the accumulated senescent cells can be selectively removed, the bio-homeostasis or the regeneration ability of the living tissue can be enhanced and thus, the chronic inflammatory response of the organism is recovered, leading to the treatment of various diseases caused by aging. Recently, it has been reported that degenerative diseases such as degenerative arthritis and cardiovascular disease can be alleviated by using drugs (senolytics) that remove senescent cells. However, as few as about 5 senolytics have been discovered so far, and the molecular mechanism of senescent cell clearance is unclear. Accordingly, there is a need to develop research on discovering drug candidates that can remove only senescent cells with various clinical values.

### DESCRIPTION OF EMBODIMENTS

### TECHNICAL PROBLEM

One aspect is to provide a compound represented by Formula 1 or Formula 2, or a pharmaceutically acceptable salt thereof: 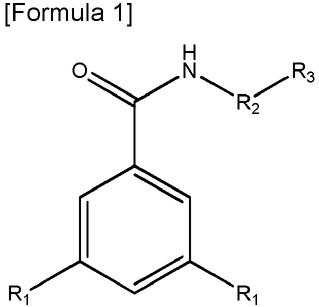 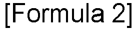 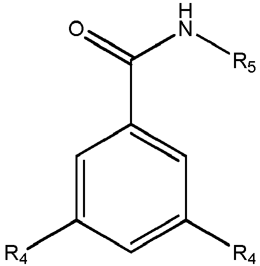 wherein, in Formula 1, R₁ is -OH or -SH, R₂ is a cationic moiety, and R₃ is a mitochondria-targeting moiety, and in Formula 2, R₄ is -OH or -SH, and R₅ is a mitochondria-targeting peptide.

Another aspect is to provide a pharmaceutical composition for preventing or treating aging-related diseases, the pharmaceutical composition including the compound or a pharmaceutically acceptable salt thereof as an active ingredient.

Another aspect is to provide a method of preventing or treating aging-related diseases, the method including administering the compound or a pharmaceutically acceptable salt thereof to a subject in need thereof.

Another aspect is to provide a use of the compound or a pharmaceutically acceptable salt thereof in the preparation of a composition for preventing or treating aging-related diseases.

### SOLUTION TO PROBLEM

One aspect is to provide a compound represented by Formula 1 or Formula 2, or a pharmaceutically acceptable salt thereof:

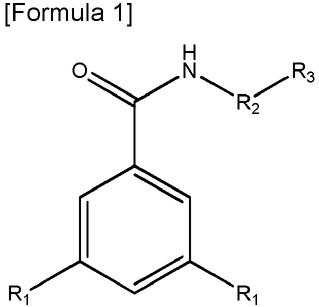

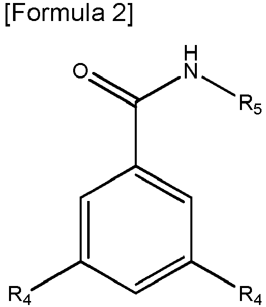

In Formula 1, R₁ may be -OH or -SH, R₂ may be a cationic moiety, and R₃ may be a mitochondria-targeting moiety, and in Formula 2, R₄ may be -OH or -SH, and R₅ may be a mitochondria-targeting peptide.

The cationic moiety may be an oxonium ion, quaternary ammonium or quaternary phosphonium.

The mitochondria-targeting moiety refers to a function of making a corresponding substance be targeted to the mitochondria inside the cell, and may be triphenylphosphonium (TPP), dequalinium, guanidinium, triethylammonium, pyridinium, 3-phenylsulfonyl furoxan, F16, 2,3-dimethylbenzothiazolium iodide, rhodamine 19, or rhodamine 123.

R₂ may be connected to amide or R₃ directly or through a linker. The linker may be an alkyl group, an alkene group, or an alkyne group, each of which has 1 to 10, 1 to 5, 1 to 4, or 2 or 3 carbon atoms, but embodiments of the present disclosure are not limited thereto. In addition, the linker may include a functional group, capable of forming an appropriate bond such as an amide bond, at one end.

The mitochondria-targeting peptide refers to a function of making a corresponding substance be targeted the mitochondria inside a cell, and may include a mitochondria targeting sequence (MTS). In one or more embodiments, the mitochondria-targeting peptide may be Leu-Leu-Arg-Ala-Ala-Leu-Arg-Lys-Ala-Ala-Leu (LLRAALRKAAL), Met-Leu-Arg-Ala-Ala-Leu-Ser-Thr-Ala-Arg-Arg-Gly-Pro-Arg-Leu-Ser-Arg-Leu-Leu (MLRAALSTARRGPRLSRLL), Met-Leu-Ser-Leu-Arg-Gln-Ser-Ile-Arg-Phe-Phe-Lys (MLSLRQSIRFFK), Leu-Ser-Arg-Thr-Arg-Ala-Ala-Ala-Pro-Asn-Ser-Arg-lle-Phe-Thr-Arg (LSRTRAAAPNSRIFTR), Met-Ile-Ala-Ser-His-Leu-Leu-Ala-Tyr-Phe-Phe-Thr-Glu-Leu-Asn (MIASHLLAYFFTELN), Met-Ile-Ala-Ser-His-Leu-Leu-Ala-Tyr-Phe-Phe-Thr-Glu-Leu-Asn (MIASHLLAYFFTELN), Lys-Leu-Ala-Lys-Leu-Ala-Lys (KLAKLAK), Lys-Leu-Ala-Lys-Arg-Gly-Asp (KLAKRGD), or Lys-Leu-Ala-Lys-Leu-Ala-Lys-Arg-Gly-Asp (KLAKLAKRGD), and is not limited as long as a peptide includes KLAK or RGD. In addition, each amino acid listed above includes a modified amino acid.

In the peptide, amino acids may be linked via a peptide bond in the order of the N-terminal and the C-terminal of an amino acid; the hydroxy group of the C-terminal carboxyl group may be substituted with one selected from an amine group, an alcohol group, an ether group, and an acetyl group; a specific chemical group may not be bonded; or other chemical groups may be chemically bonded, for example, an amine group may be bonded.

In the present disclosure, due to the mitochondria-targeting moiety or the mitochondria-targeting peptide, the compound according to the present disclosure may be targeted to the mitochondria inside the cell, and the compound may enter the mitochondria by passing through the double membrane of the mitochondria.

In the present disclosure, the compound may enter the mitochondria due to the mitochondria-targeting moiety or the mitochondria-targeting peptide, and once the compound enters the mitochondria, the compound may form, specifically to the senescent cell, a macromolecule inside the mitochondria of the senescent cell to induce apoptosis.

In an embodiment, the compound represented by Formula 1 may be represented by Formula 3.

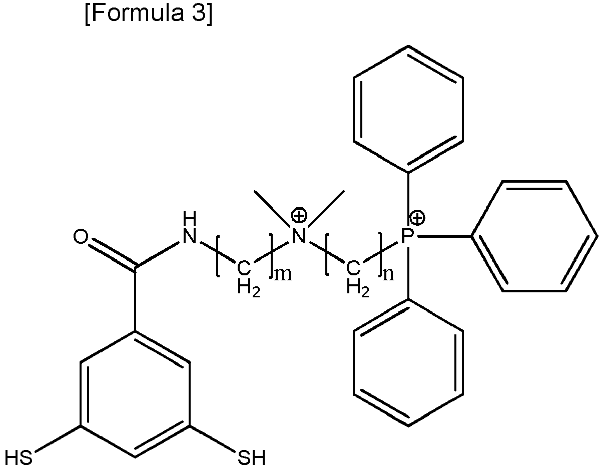

Here, in Formula 3, m and n may be 1 to 20, respectively.

In an embodiment, the compound represented by Formula 2 may be one selected from compounds represented by Formulae 4 to 7:

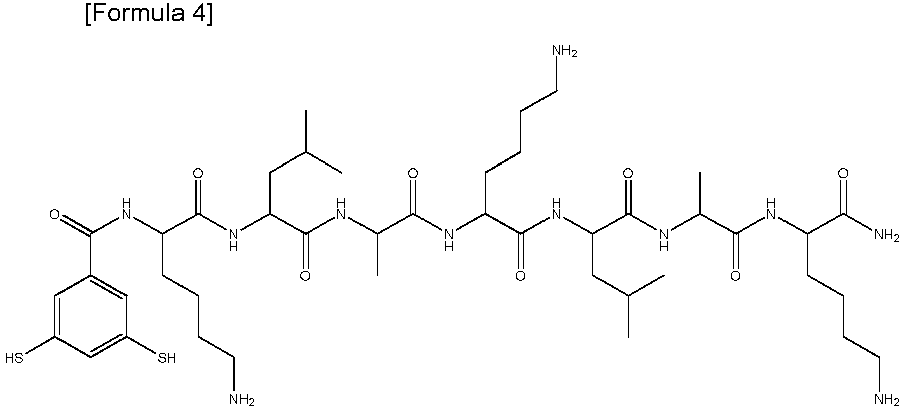

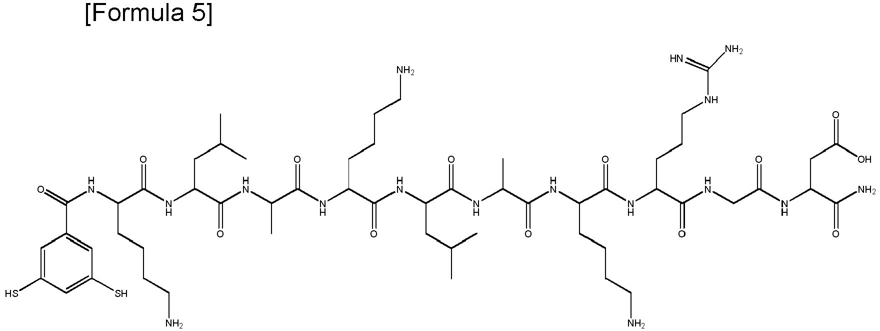

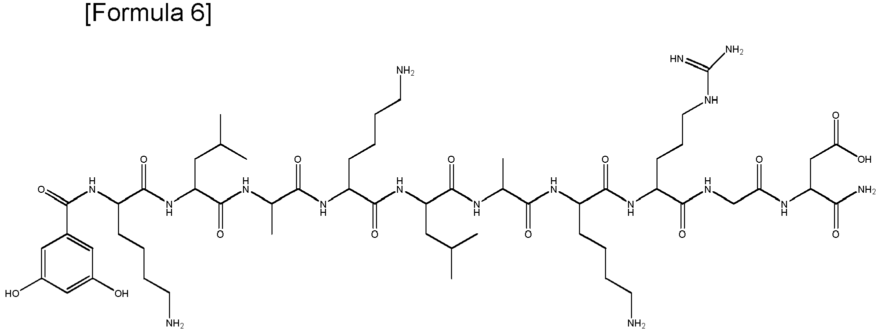

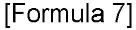

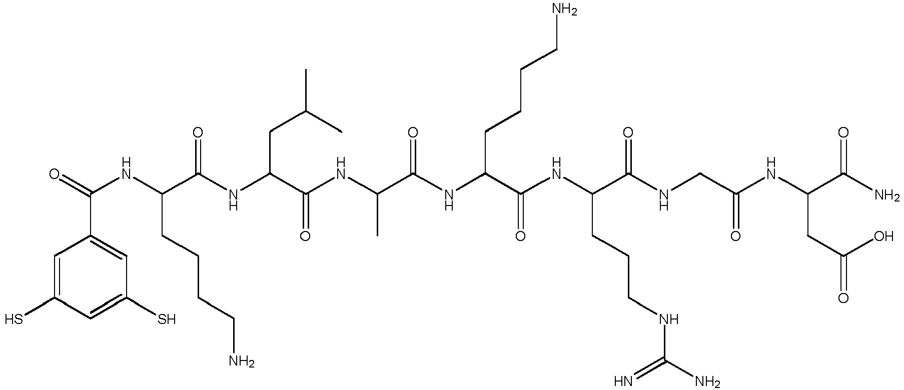

R₁ of Formula 1 or R₄ of Formula 2 may be oxidized to form a bond with a plurality of other compounds. In an embodiment, when R₁ or R₄ is -SH, R₁ and R₄ may be oxidized to form a disulfide bond, -S-S-, with another compound, and when R₁ or R₄ is -OH, R₁ and R₄ may be oxidized to form a peroxide bond, -O-O-, with other compounds. Through the bondings, the compound may be linked to other compounds to form a macromolecule.

In addition, the oxidation may occur due to reactive oxygen species (ROS).

In the present disclosure, the compound is oxidized by ROS over-expressed in senescent cells compared to normal cells to form macromolecules in the mitochondria of senescent cells, thereby disrupting the integrity of the mitochondrial membrane and selectively inducing apoptosis of senescent cells.

Another aspect is to provide a pharmaceutical composition for preventing or treating aging-related diseases including the compound or a pharmaceutically acceptable salt thereof as an active ingredient.

The aging-related disease may be one or more selected from senile cardiovascular disease and disorder, senile lung disease, idiopathic pulmonary fibrosis, chronic obstructive pulmonary disease, osteoarthritis, senile eye disease, and skin aging, and the senile eye disease may be senile macular degeneration. In addition, the macular degeneration includes dry-type macular degeneration and wet-type macular degeneration.

In an embodiment, the compound of the composition may induce apoptosis in mitochondria in senescent cells.

In the present disclosure, the compound of the composition may enter the mitochondria due to the mitochondria-targeting moiety or the mitochondria-targeting peptide, and in particular, the compound is oxidized by ROS over-expressed in senescent cells compared to normal cells to form a macromolecule in the mitochondria of senescent cells, and thus, the integrity of the mitochondrial membrane is disrupted and apoptosis of senescent cells may be selectively induced.

The apoptosis is a form of cell death controlled by genes. The apoptosis is distinguished from necrosis, which is a necrosis or pathological death of cells, and is the death of cells caused by stimulation such as burns, bruises, and poisons. In the apoptosis, the cell death starts with the shrinking of the cell, and then a gap is created between adjacent cells, and within the cell, DNA is regularly cleaved and fragmented.

The compound may exist in the form of a pharmaceutically acceptable salt. The pharmaceutically acceptable salt includes an acid or base addition salt and a stereochemically isomeric form thereof, and may be, for example, an addition salt of an organic acid or an inorganic acid. The salt includes any salt that maintains the activity of the parent compound in the administration target and does not cause undesirable effects, and is not particularly limited.

These salts include inorganic and organic salts, for example, acetic acid, nitric acid, aspartic acid, sulfonic acid, sulfuric acid, maleic acid, glutamic acid, formic acid, succinic acid, phosphoric acid, phthalic acid, tannic acid, tartaric acid, hydrobromic acid. , propionic acid, benzenesulfonic acid, benzoic acid, stearic acid, lactic acid, bicarbonic acid, bisulfuric acid, bitartric acid, oxalic acid, butyric acid, calcium idete, carbonyl acid, chlorobenzoic acid, citric acid, idetic acid, toluene sulfonic acid, fumaric acid, gluceptic acid, esylic acid, pamoic acid, gluconic acid, methylnitric acid, malonic acid, hydrochloric acid, hydroiodoic acid, hydroxynaphtholic acid, isethionic acid, lactobionic acid, mandelic acid, mucous acid, naphthoic acid, muconic acid, p-nitromethane sulfonic acid, hexamic acid, pantothenic acid, monohydrogen phosphate, dihydrogen phosphate, salicylic acid, sulfamic acid, sulfanilic acid, methanesulfonic acid.

In addition, the form of the salt may include salts of alkali and alkaline earth metals, such as ammonium salt, lithium salt, sodium salt, potassium salt, magnesium salt, or calcium salt, for example, an organic base-containing salt, such as benzathine, N-methyl-D-glucamine, or a hydrabamine salt, and a salt having an amino acid such as arginine or lysine. In addition, the salt form may also be converted to the free form by treatment with a suitable base or acid.

The term "therapeutic agent" or "pharmaceutical composition" refers to a molecule or compound that imparts several beneficial effects when administered to a subject. The beneficial effects include enabling diagnostic decisions; alleviation of disease, symptom, disorder, or condition; reducing or preventing the onset of disease, symptom, disorder, or condition; and, in general, the response to disease, symptom, disorder, or condition.

The pharmaceutical composition of the present disclosure may be in any form suitable for the intended method of administration. In the pharmaceutical composition of the present disclosure, "administration" refers to introducing a certain substance to the patient by any suitable method, and the administration route of the pharmaceutical composition is any general route as long as the drug can reach the target tissue. For example, intraperitoneal administration, intravenous administration, intramuscular administration, subcutaneous administration, intradermal administration, oral administration, topical administration, intranasal administration, intrapulmonary administration, rectal administration, etc. may be used, but the administration route is not limited thereto. In addition, the pharmaceutical composition of the present disclosure may be administered by any device capable of moving the active ingredient to target cells.

Another aspect is to provide a method of preventing or treating aging-related diseases, including administering the compound or a pharmaceutically acceptable salt thereof to a subject in need thereof.

Another aspect is to provide a use of the compound or a pharmaceutically acceptable salt thereof for use in the preparation of a composition for preventing or treating aging-related diseases.

The pharmaceutical composition of the present disclosure may be administered orally or parenterally. In an embodiment, the pharmaceutical composition may be administered orally. When administered parenterally, the pharmaceutical composition of the present disclosure may be administered by intravenous injection, subcutaneous injection, intramuscular injection, or intraperitoneal injection. The route of administration of the pharmaceutical composition of the present disclosure may be determined according to the type of disease to be applied.

The pharmaceutical composition provided herein may be formulated according to a conventional method into oral dosage forms such as powders, granules, tablets, capsules, suspensions, emulsions, syrups, or aerosols, or parenteral formulations such as suspensions, emulsions, freeze-dried preparations, external preparations, suppositories, sterile injection solutions, or implantable preparations.

The pharmaceutical composition may further include, in addition to the active ingredient (that is, the compound or a pharmaceutically acceptable salt thereof), a pharmaceutically acceptable excipient that can be used for formulation.

Excipients that can be used in the formulation of the pharmaceutical composition of the present disclosure may be selected from carriers, vehicles, diluents, solvents, for example, monohydric alcohols such as ethanol, isopropanol, or polyhydric alcohols such as glycerol and edible oils such as soybean oil, coconut oil, olive oil, safflower oil, cottonseed oil, oily esters such as ethyl oleate, isopropyl myristate; binders, adjuvants, solubilizers, thickeners, stabilizers, disintegrants, lubricants, lubricants, buffers, emulsifiers, wetting agents, suspending agents, sweetening agents, coloring agents, flavoring agents, coating agents, preservatives, antioxidants, processing agents, drug delivery modifiers, and enhancers such as calcium phosphate, magnesium state, talc, monosaccharides, disaccharides, starch, gelatin, cellulose, methylcellulose, sodium carboxymethyl cellulose, dextrose, hydroxypropyl-β-cyclodextrin, polyvinylpyrrolidone, low melting point wax, and ion exchange resin, but are not limited thereto.

Pharmaceutically acceptable carriers included in the pharmaceutical composition of the present disclosure may be any carries that are commonly used in formulation, and include lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oil, but are not limited thereto. The pharmaceutical composition of the present disclosure may further include, in addition to the components described above, a lubricant, a wetting agent, a sweetening agent, a flavoring agent, an emulsifying agent, a suspending agent, a preservative, and the like. Suitable and pharmaceutically acceptable carriers and formulations are described in detail in Remington's Pharmaceutical Sciences (19th ed., 1995).

The pharmaceutical composition may be formulated in the form of various oral dosage forms. For example, the pharmaceutical composition may be formulated into any oral dosage form, such as tablets, pills, hard/soft capsules, liquids, suspensions, emulsifiers, syrups, granules, or elixirs. Depending on the usual composition of each formulation, the oral dosage form may include, in addition to the active ingredient, a pharmaceutically acceptable carrier, for example, a diluent such as lactose, dextrose, sucrose, mannitol, sorbitol, cellulose, and/or glycine, a lubricant such as silica, talc, stearic acid and a magnesium or calcium salt thereof, and/or polyethylene glycol.

In addition, when the oral dosage form is a tablet, the oral dosage form may contain a binder, for example, magnesium aluminum silicate, starch paste, gelatin, tragacanth, methylcellulose, sodium carboxymethylcellulose, and/or polyvinylpyrrolidine, and in some cases, the oral dosage form may contain a disintegrant such as starch, agar, alginic acid, or sodium salt thereof, a boiling mixture and/or an absorbent, a colorant, a flavoring agent, or a sweetening agent.

In addition, the pharmaceutical composition may be formulated in a parenteral dosage form, in which case the pharmaceutical composition may be administered by a parenteral administration method such as subcutaneous injection, intravenous injection, intramuscular injection, or intrathoracic injection. At this time, in order to formulate the pharmaceutical composition into the parenteral dosage form, the pharmaceutical composition may be prepared as a solution or suspension in which the active ingredient is mixed in water with a stabilizer or a buffer, and such a solution or suspension may be prepared in a unit dosage form of an ampoule or vial.

In addition, the pharmaceutical composition may be sterilized or may further include adjuvants such as preservatives, stabilizers, hydrating agents or emulsification accelerators, salts and/or buffers for controlling osmotic pressure, or may further include other therapeutically useful substances, and may be formulated according to a conventional method of mixing, granulating or coating.

The amount of the compound or a pharmaceutically acceptable salt thereof in the pharmaceutical composition may be appropriately adjusted depending on the purpose of use of the pharmaceutical composition, the form of the formulation, etc. In an embodiment, the amount thereof may be, based on the total weight of the pharmaceutical composition, from 0.001 wt% to 99 wt%, 0.001 wt% to 90 wt%, 0.001 wt% to 50 wt%, 0.01 wt% to 50 wt%, 0.1 wt% to 50 wt%, or 1 wt% to 50 wt%, but is not limited thereto.

In addition, the therapeutically effective amount of the compound or a pharmaceutically acceptable salt thereof included in the pharmaceutical composition of the present disclosure refers to an amount required for administration in order to expect a disease treatment effect. Therefore, the therapeutically effective amount may be adjusted according to the patient's disease type, the severity of the disease, the type of the active ingredient to be administered, the type of formulation, the age, sex, weight, or health condition of the patient, diet, and the administration time and administration method of the drug. For example, to mammals, including humans, a pharmaceutically effective amount of 0.01 mg/kg to 500 mg/kg (body weight) per day may be administered. The pharmaceutically effective amount may depend on an amount capable of obtaining a target effect, for example, a therapeutic and/or prophylactic effect for an aging-related disease. The pharmaceutically effective amount may be administered all at once per day or divided into two or more fractions, each of which is administered separately through an oral or parenteral route (for example, intravenous injection, intramuscular injection, etc.).

The pharmaceutical composition may be used to treat an aging-related disease by including administering the pharmaceutical composition to the subject in an amount effective to prevent or treat an aging-related disease.

The subject may be a mammal. The mammal may be a human, dog, cat, cow, goat, or pig.

The terms "treatment" or "treating" or "relaxing" or "improving" may used interchangeably. These terms include a therapeutic benefit and/or a prophylactic benefit, but are not limited thereto. For example, these terms may refer to a method of obtaining an advantageous or desired result. A therapeutic benefit refers to any therapeutically significant improvement of one or more diseases, disorders or symptoms under treatment or effects thereon. Regarding a prophylactic benefit, the composition may be administered to a subject at risk of developing a particular disease, condition, or symptom, or to a subject reporting one or more physiological symptoms of the disease, even if the disease, condition, or symptom is not yet present.

The term "effective amount" or "therapeutically effective amount" refers to an amount of an agent sufficient to produce an advantageous or target result. The therapeutically effective amount may vary according to one or more of the subject and condition to be treated, the weight and age of the subject, the severity of the condition, the mode of administration, and the like, which can be easily determined by a person skilled in the art. In addition, the terms are applied to the dosage to provide an image for detection by any of the imaging methods described herein. The specific dosage may vary depending on one or more of the particular agent selected, the dosage regimen that follows, whether being administered in combination with other compounds, the timing of administration, the tissue being imaged, and the body delivery system carrying the same.

The terms and methods described for the above disclosure are applied equally to each of the disclosures.

### ADVANTAGEOUS EFFECTS OF DISCLOSURE

A compound according to an aspect or a pharmaceutically acceptable salt thereof, and a pharmaceutical composition containing the same can specifically induce apoptosis of senescent cells and can be effectively used for the prevention or treatment of aging-related diseases.

### BRIEF DESCRIPTION OF DRAWINGS

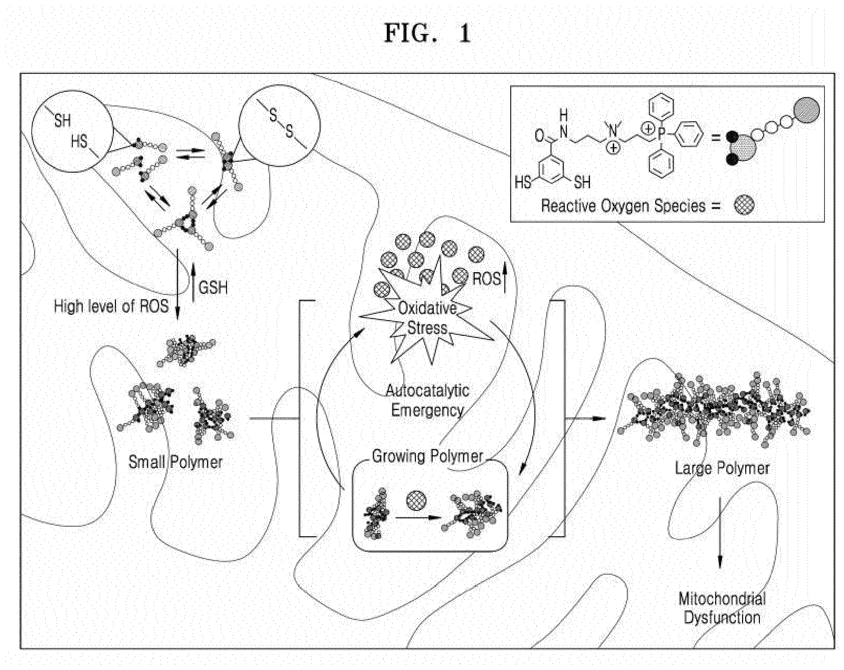

FIG. 1 shows a diagram schematically illustrating the apoptosis mechanism of the present disclosure.
FIG. 2 shows a chemical structure of SP-101 according to an embodiment of the present disclosure.
FIG. 3 shows a diagram schematically showing that the thiol group of the present disclosure is oxidized to form a disulfide bond.
FIG. 4 shows scanning electron microscope (SEM) and transmission electron microscopy (TEM) images in which SP-101 is oxidized by ROS to form macromolecules.
FIG. 5 shows a graph showing the ROS production of HeLa, IMR90, and senescent cells.
FIG. 6 shows a graph showing the cytotoxicity of SP-101.
FIG. 7 shows photographs of senescent cells observed under a microscope showing that senescent cells are reduced by SP-101.
FIG. 8 shows a photograph of the result of confirming the effect of SP-101 inhibiting the generation of choroidal neovascularization.
FIG. 9 shows a graph of the result of confirming the effect of SP-101 inhibiting the generation of choroidal neovascularization.
FIG. 10 shows a chemical structure of SH-KLAKLAKRGD according to an embodiment of the present disclosure.
FIG. 11 shows a graph and TEM image showing the molecular size of SH-KLAKLAKRGD to form a macromolecule.
FIG. 12 is a graph showing the cytotoxicity of SH-KLAKLAKRGD:
   FIG. 12A is a graph showing the cytotoxicity of SH-KLAKLAKRGD in senescent cells, and FIG.12B is a graph showing the cytotoxicity of SH-KLAKLAKRGD in normal cells.

### MODE OF DISCLOSURE

Hereinafter, the present disclosure will be described in more detail through Examples. However, these examples are for illustrative purposes only, and the scope of the present disclosure is not limited to these examples.

### Example 1. Synthesis and analysis of SP-101, a mitochondria-targeting compound

SP-101, a mitochondria-targeting compound having a targeting ability specific to mitochondria, was prepared.

Specifically, 0.8 g of methyl 3,5-dihydroxybenzoate, 0.2 g of dimethylcarbamothioic chloride, and 0.2 g of DABCO were dissolved in 100 mL of DMF, and stirred at room temperature for 24 hours. The mixture was purified by HPLC to obtain white powder. 0.5 g of the resulting methyl 3,5-bis((dimethylcarbamothioyl)oxy)benzoate was dissolved in 10 mL of diphenyl ether and stirred under reflux for 24 hours. The resultant mixture was purified by HPLC to obtain yellow powder. 0.2 g of the obtained methyl 3,5-bis((dimethylcarbamoyl)thio)benzoate and 0.1 g of KOH were dissolved in 10 ml of methanol and 10 ml of water, and stirred under reflux for 24 hours. The mixture was purified by HPLC. 0.2 g of the obtained 3,5-dimercaptobenzoic acid and 2 g of Ph₃CCl were dissolved in 10 ml of methanol and stirred under reflux for 24 hours. The mixture was purified by HPLC. 0.1 g of the obtained 3,5-bis(tritylthio)benzoic acid), 0.1 g of (3-((3-aminopropyl)dimethylammonio)propyl)triphenylphosphonium), and 0.1 g of EDC were dissolved in 10 ml of methanol and stirred at room temperature for 24 hours. The mixture was purified by HPLC. 0,1 g of the obtained (3-((3-(3,5-bis(tritylthio)benzamido)propyl)(metheyliumyl)(methyl)ammonio)propyl)triphenylphos phonium, 0.05 g of TIPS, and 0.05 g of TFA were dissolved in 10 ml of DCM and stirred at room temperature for 24 hours. The resultant mixture was purified by HPLC.

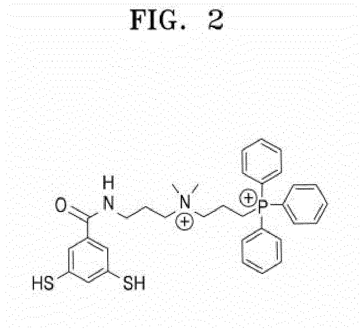

As a result, (3-((3-(3,5-dimercaptobenzamido)propyl)dimethylammonium)propyl)triphenylphosphonium was obtained, which is hereinafter referred to as 'SP-101' (FIGS. 2).

FIG. 2 shows a chemical structure of SP-101 according to an embodiment of the present disclosure.

### Example 2. Synthesis and analysis of SH-KLAKLAKRGD, a mitochondria-targeting compound

SH-KLAKLAKRGD, a mitochondria-targeting compound having a targeting ability specific to mitochondria, was prepared by solid phase peptide synthesis.

Specifically, 200 mg MBHA amide resin was used, and the first amino acid loading capacity was 0.106 mmol.

First, 200 mg MBHA amide resin was put into a syringe with a filter inserted and swelled in DMF for 30 minutes. Then, the fluorenylmethoxycarbonyl protecting group (Fmoc) was removed therefrom for 50 minutes by using 3 ml of 20% piperidine in DMF (Fmoc-deprotection). The result was washed with DMF and DCM, three times for each. A D amino acid solution (5 eq amino acid, 5 eq 0.5M HBTU, and 10 eq DIPEA) was added, followed by D amino acid coupling for 1 hour and 50 minutes. The result was washed with DMF and DCM, three times for each. In the same way, in this stated order, a G amino acid, a R amino acid, a R amino acid without deprotection, a K amino acid, an A amino acid, a L amino acid, a K amino acid, an A amino acid, a L amino acid, and a K amino acid were subjected to coupling. At this time, in the case of Fmoc-Arg(pbf)-OH, the amino acid coupling process was performed once again without the deprotection process (#2, #3). After the synthesis of NH2-KLAKLAKRGD was completed, 2 eq disulfide 4, 2 eq HBUT, and 4 eq DIPEA were dissolved in DMF and reacted for 24 hours or more. The result was washed with DMF and DCM, three times for each. Reagent R (4.5 ml of TFA, 0.25 ml of thioanisole, 0.15 ml of 1,2-ethanedithiol, and 0.1 ml of anisole) was used as a peptide cleavage cocktail, and the cleavage process was performed for 2 hours and 30 minutes. The cleavage solution was precipitated in 40 ml of cold ether. After recovering the precipitate by centrifugation, the precipitate was washed once more with 45 ml of cold ether. The obtained crude was dried, and then, a methanol solution having a concentration of 20 mg/ml was prepared. The crude was separated using HPLC, and a C18-reverse phase column was used.

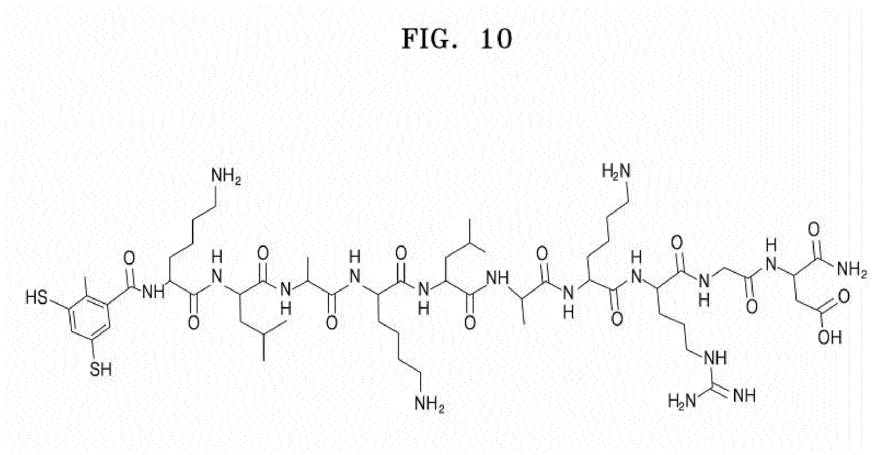

As a result, as shown in FIG. 10, SH-KLAKLAKRGD was obtained.

FIG. 10 shows a chemical structure of SH-KLAKLAKRGD according to an embodiment of the present disclosure.

### Experimental Example 1. Confirmation of macromolecular formation of SP-101 by ROS

It was confirmed that SP-101 was oxidized by ROS to form macromolecules.

Specifically, 1 mM SP-101 and 10 mM SP-101 were each dissolved in water and stirred for 24 hours. A drop of an aqueous solution of SP-101 was placed on a formvar/carbon-coated copper grid and evaporated under atmospheric conditions. The sample was stained with a 2 wt% uranyl acetate solution, evaporated for 1 minute, and then the excess solution was removed therefrom by using a filter paper. The specimens were observed with a JEM-1400 transmission electron microscopy (TEM) and a scanning electron microscopy (SEM), each operating at 120 kV.

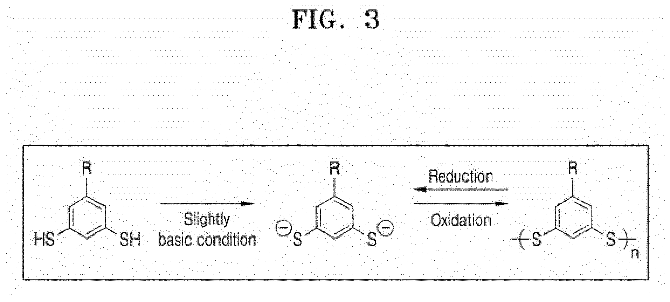

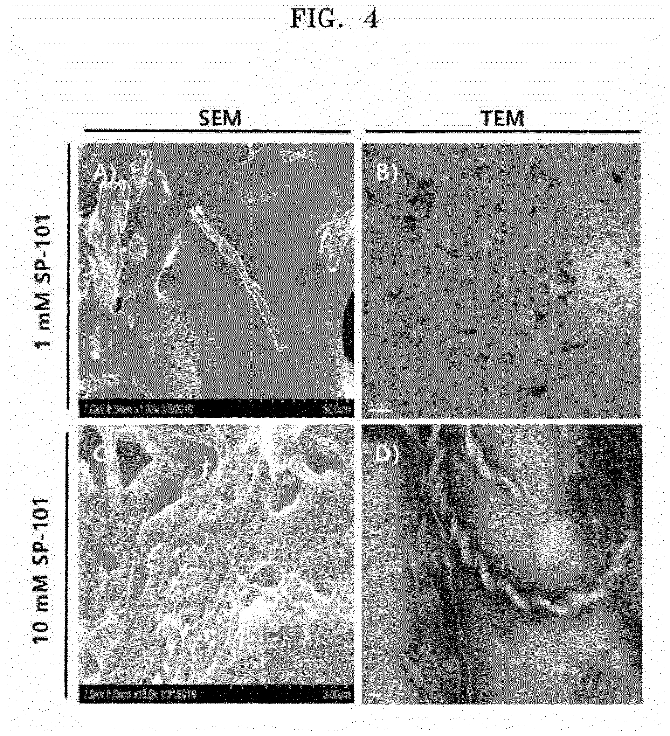

As a result, it was confirmed that SP-101 was oxidized to form a macromolecule (FIGS. 3 and 4).

FIG. 3 shows a diagram schematically showing that the thiol group of the present disclosure is oxidized to form a disulfide bond.

FIG. 4 shows scanning electron microscope (SEM) and transmission electron microscopy (TEM) images in which SP-101 is oxidized by ROS to form macromolecules.

### Experimental Example 2. Confirmation of ROS over-expression in senescent cells

It was confirmed whether ROS was over-expressed in senescent cells compared to other cells.

Specifically, HeLa cells, IMR90 cells, and aged chondrocyte cells were seeded at a density of 4X10⁴ cells/100 µL per well on a Lab Tek II slide chamber in DMEM (Life Technologies) supplemented with 10% FBS and 1% penicillin/streptomycin. Then, the cells were incubated overnight under conditions of 5% CO₂ and 37 °C. Subsequently, by using the Cellular ROS assay kit, the cell plate was treated with100 µL/well of a red working solution (ROS). The fluorescence increase was monitored at Ex/Em = 520/605 nm (590 nm was blocked) using the bottom reading mode.

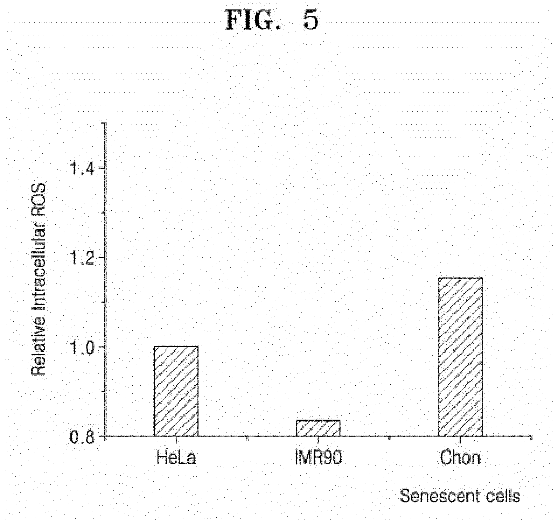

As a result, it was confirmed that ROS was over-expressed in senescent cells compared to HeLa and IMR90 cells (FIG. 5).

FIG. 5 shows a graph showing the ROS production of HeLa, IMR90, and senescent cells.

### Experimental Example 3. Confirmation of cytotoxicity of SP-101

Cytotoxicity was analyzed to observe the killing effect of SP-101 on senescent cells.

Specifically, the cell viability of IMR90 and senescent cells against SP-101 was evaluated by identifying the reduction of 3-(4,5-dimethylthiazol-2-yl)-2,5-diphenyltetrazolium bromide (MTT) to insoluble formazan. Cells were seeded at a density of 5×10³ cells per well in a 96-well plate and cultured overnight, followed by treatment with different concentrations of SP-101 in DMEM medium containing 10% FBS and cultured for 24 hours. Then, the cells were cultured with MTT, and then the crystallized formazan was quantified by measuring the absorbance thereof at 595 nm by using an ELISA plate reader. Results were expressed as percent viability = [(A550 (treated cells)-background value)/(A550 (untreated cells)-background value)] x 100.

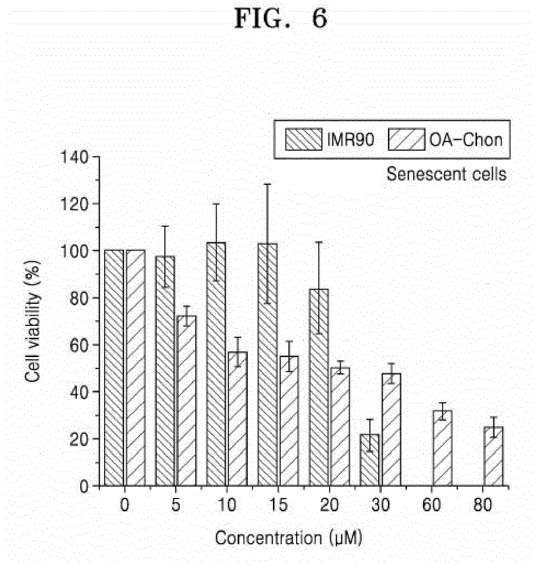

As a result, it was confirmed that in the concentration range of 5 µM to 30 µM of SP-101, more senescent cells were killed than normal cells, IMR90 (FIG. 6).

These results indicate that SP-101 kills senescent cells more specifically and effectively than normal cells.

FIG. 6 shows a graph showing the cytotoxicity of SP-101.

### Experimental Example 4. Confirmation of reduction in senescent cells due to SP-101

It was confirmed under a microscope that senescent cells were reduced by SP-101.

Specifically, SA-b-galactosidase staining was used. Cells that were not treated with anything were used as a control, and those treated with 30 µM of SP-101 were used as an experimental group. SA-gal staining was performed using a kit (Biovision, K320-250) according to the manufacturer's instructions. Senescent cells were identified as blue-stained cells under an optical microscope.

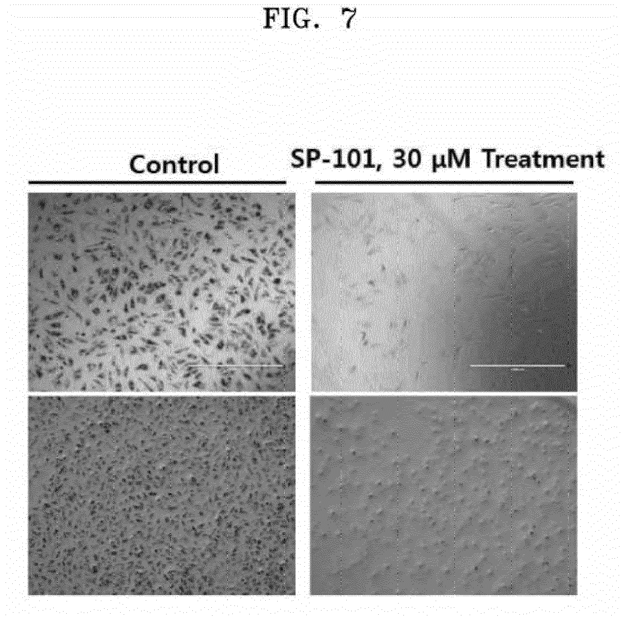

As a result, it was confirmed that senescent cells were reduced in the experimental group treated with SP-101 (FIG. 7).

Taking the above results together, it can be seen that phenyldithiol of SP-101 was oxidized by ROS over-expressed in senescent cells to form macromolecules, thereby disrupting the mitochondrial membrane and specifically killing senescent cells.

FIG. 7 shows photographs of senescent cells observed under a microscope showing that senescent cells were reduced by SP-101.

### Experimental Example 5. Confirmation of the inhibitory effect of SP-101 on choroidal neovascularization

The CNV model was used to confirm whether SP-101 has an effect of inhibiting the production of choroidal neovascularization.

Specifically, the mice were anesthetized by intraperitoneal injection of ketamine hydrochloride and xylazine hydrochloride. By using a diode laser with a wavelength of 810 nm, eight photocoagulation spots, each having the size of 75 µm, the intensity of 250 mW, and the time of 50 ms, were made in concentric circles among the main retinal vessels around the optic nerve. The rupture of the Bruck's membrane was confirmed by identifying that cavitation bubbles had been formed in the choroid. Immediately after laser irradiation, the experimental groups treated with 10 µM, 50 µM, and 100 µM of SP-101, and phosphate buffered saline and Eylea, which were used as control groups, were injected into the vitreous cavity. Two weeks after laser irradiation, fluorescein angiography was performed thereon using a confocal scanning laser fundus camera. After fluorescein-dextran was rapidly injected intravenously, images thereof were taken at the beginning (2 min) and late (10 min).

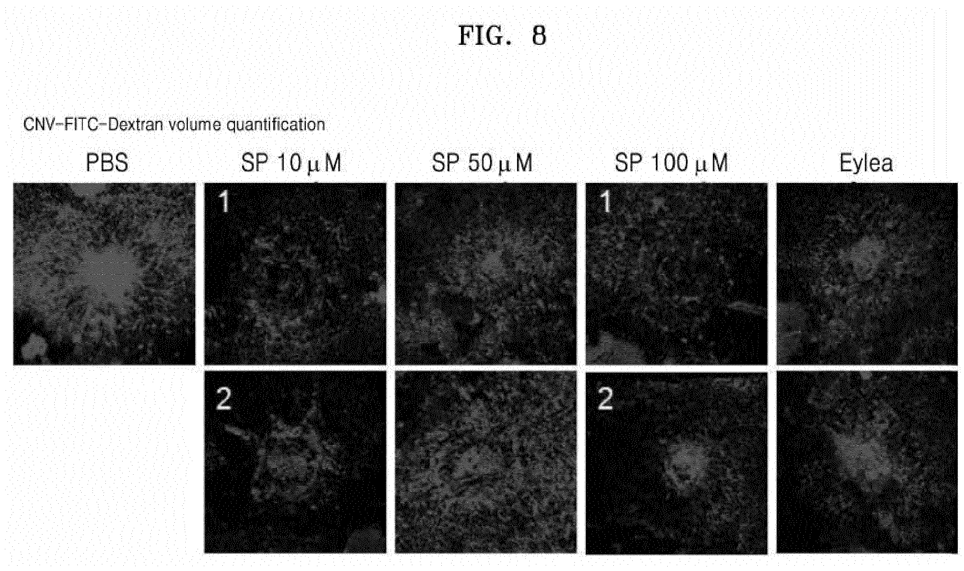

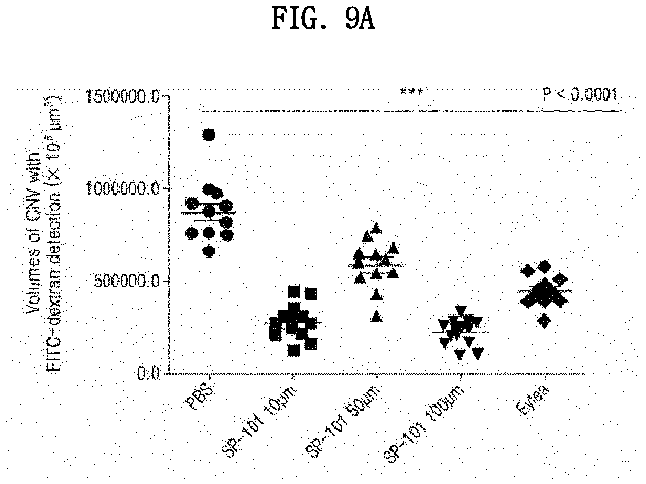

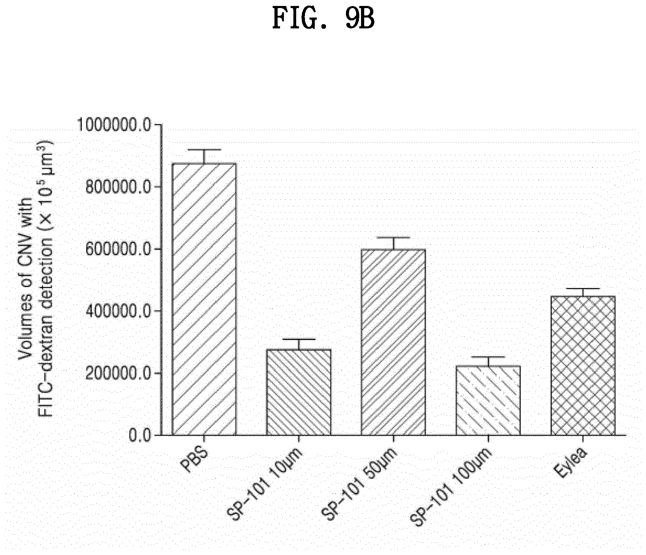

As a result, compared with the volume of choroidal neovascularization, it was identified that the volume of the groups treated with 10 µM and 100 µM of SP-101 was decreased by about twice compared to the Eylea control group (FIGS. 8 and 9).

These results mean that SP-101 eliminates senescent cells and factors secreted by senescent cells are thus removed, thereby inhibiting the occurrence of new blood vessels, and SP-101 is effective about twice as effective as Eylea, so choroidal neovascularization was effectively inhibited.

FIG. 8 shows a photograph of the result of confirming the effect of SP-101 inhibiting the generation of choroidal neovascularization.

FIG. 9 shows a graph of the result of confirming the effect of SP-101 inhibiting the generation of choroidal neovascularization.

### Experimental Example 6. Confirmation of self-assembly by formation of SH-KLAKLAKRGD

SH-KLAKLAKRGD (1 mM and 10 mM) was dissolved in water and stirred for 24 hours. A drop of SH-KLAKLAKRGD aqueous solution was placed on a formvar/carbon coated copper grid and allowed to evaporate at ambient conditions. The sample was stained with 2 wt% uranyl acetate solution and evaporated for 1 minute, and the excess solution was removed with filter paper. The specimen was observed using a JEM-1400 TEM operating at 120 kV.

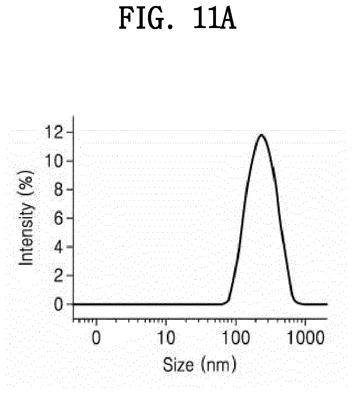

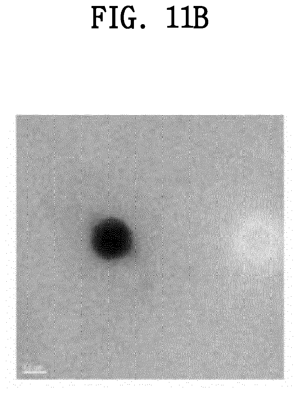

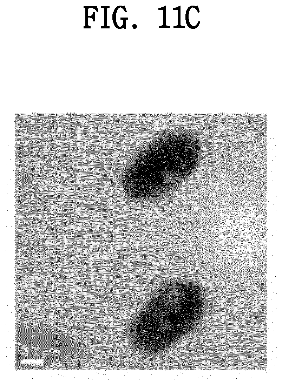

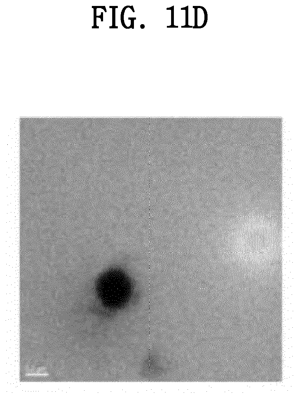

As a result, as shown in FIG. 11, the size of the polymerized SH-KLAKLAKRGD was 350 ± 100 nm, and the TEM image showed a macromolecule in which peptides were polymerized.

FIG. 11 shows a graph and TEM image showing the molecular size of SH-KLAKLAKRGD to form a macromolecule.

### Experimental Example 7. Confirmation of cytotoxicity of SH-KLAKLAKRGD

Cytotoxicity was analyzed to observe the killing effect of SH-KLAKLAKRGD on senescent cells.

Specifically, the cell viability of IMR90 and senescent cells against SH-KLAKLAKRGD was evaluated by identifying the reduction of MTT to insoluble formazan. Cells were seeded at a density of 5×10³ cells per well in a 96-well plate and cultured overnight, followed by treatment with different concentrations of SH-KLAKLAKRGD in DMEM medium containing 10% FBS and cultured for 24 hours. Then, the cells were cultured with MTT, and then the crystallized formazan was quantified by measuring the absorbance thereof at 595 nm by using an ELISA plate reader. Results were expressed as percent viability = [(A550 (treated cells)-background value)/(A550 (untreated cells)-background value)] x 100.

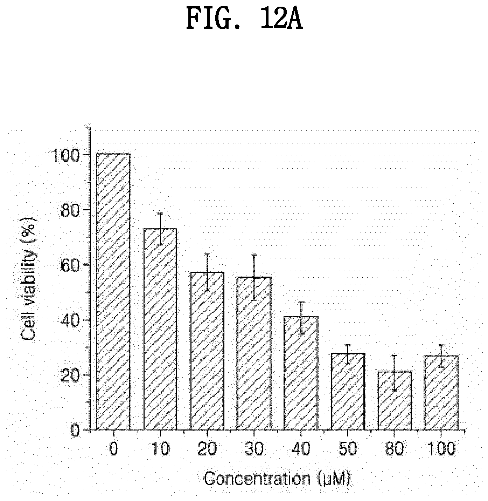

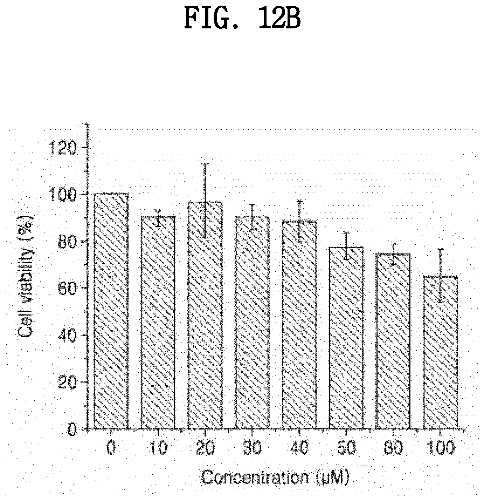

As a result, it was confirmed that the cell viability in senescent cells was lower than that in IMR90, which is a normal cell (FIG. 12).

These results indicate that SH-KLAKLAKRGD kills senescent cells more specifically and effectively than normal cells.

FIG. 12 is a graph showing the cytotoxicity of SH-KLAKLAKRGD:
FIG. 12A is a graph showing the cytotoxicity of SH-KLAKLAKRGD in senescent cells, and FIG.12B is a graph showing the cytotoxicity of SH-KLAKLAKRGD in normal cells.

## Claims

1. A compound represented by Formula 1 or Formula 2, or a pharmaceutically acceptable salt thereof: 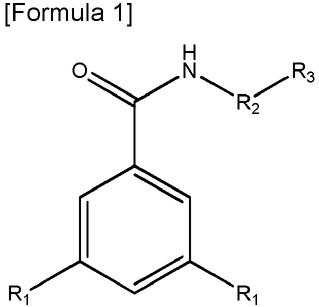 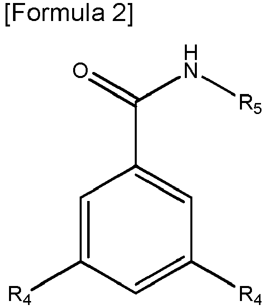 wherein, in Formula 1, R₁ is -OH or -SH, R₂ is a cationic moiety, and R₃ is a mitochondria-targeting moiety, and in Formula 2, R₄ is -OH or -SH, and R₅ is a mitochondria-targeting peptide.

2. The compound or pharmaceutically acceptable salt thereof of claim 1, wherein the cationic moiety is an oxonium ion, quaternary ammonium, or quaternary phosphonium.

3. The compound or pharmaceutically acceptable salt thereof of claim 1, wherein the mitochondria-targeting moiety is triphenylphosphonium (TPP), dequalinium, guanidinium, triethylammonium, pyridinium, 3-phenylsulfonyl furoxan, F16, 2,3-dimethylbenzothiazolium iodide, rhodamine 19, or rhodamine 123.

4. The compound or pharmaceutically acceptable salt thereof of claim 1, wherein R₂ is connected to amide or R₃ directly or through a linker.

5. The compound or pharmaceutically acceptable salt thereof of claim 1, wherein the mitochondria-targeting peptide is Leu-Leu-Arg-Ala-Ala-Leu-Arg-Lys-Ala-Ala-Leu (LLRAALRKAAL), Met-Leu-Arg-Ala- Ala-Leu-Ser-Thr-Ala-Arg-Arg-Gly-Pro-Arg-Leu-Ser-Arg-Leu-Leu (MLRAALSTARRGPRLSRLL), Met-Leu-Ser-Leu-Arg-Gln-Ser-Ile-Arg -Phe-Phe-Lys (MLSLRQSIRFFK), Leu-Ser-Arg-Thr-Arg-Ala-Ala-Ala-Pro-Asn-Ser-Arg-Ile-Phe-Thr-Arg (LSRTRAAAPNSRIFTR), Met-Ile-Ala -Ser-His-Leu-Leu-Ala-Tyr-Phe-Phe-Thr-Glu-Leu-Asn (MIASHLLAYFFTELN), Met-Ile-Ala-Ser-His-Leu-Leu-Ala-Tyr-Phe-Phe- Thr-Glu-Leu-Asn (MIASHLLAYFFTELN), Lys-Leu-Ala-Lys-Leu-Ala-Lys (KLAKLAK), Lys-Leu-Ala-Lys-Arg-Gly-Asp (KLAKRGD), or Lys-Leu-Ala - Lys-Leu-Ala-Lys-Arg-Gly-Asp (KLAKLAKRGD).

6. The compound or pharmaceutically acceptable salt thereof of claim 1, wherein the compound represented by Formula 1 is represented by Formula 3: 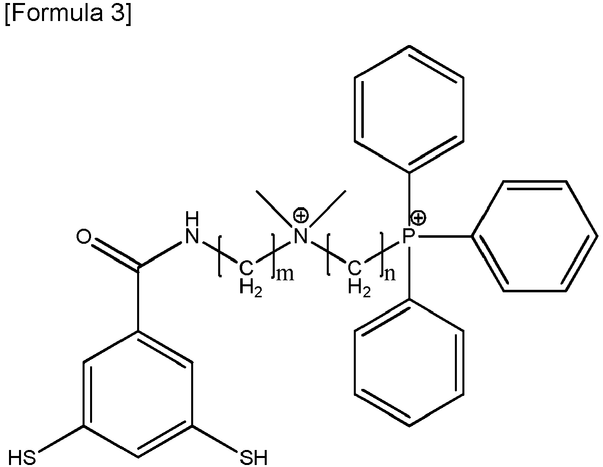 wherein, in Formula 3, m and n are each from 1 to 20.

7. The compound or pharmaceutically acceptable salt thereof of claim 1, wherein the compound represented by Formula 2 is one selected from compounds represented by Formulae 4 to 7:

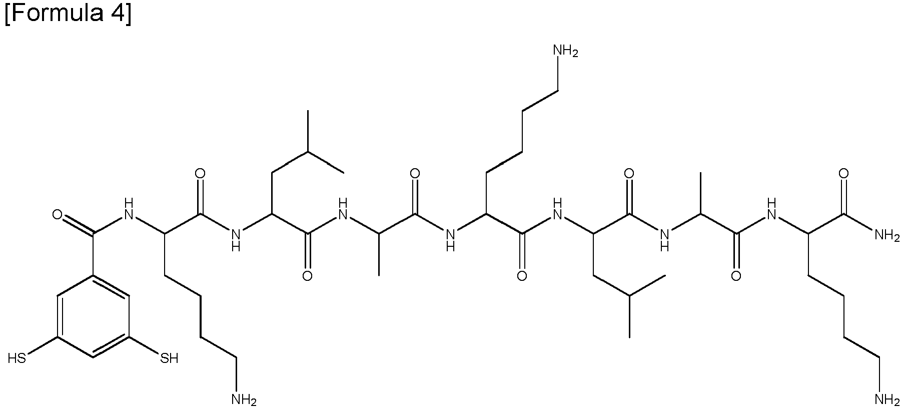

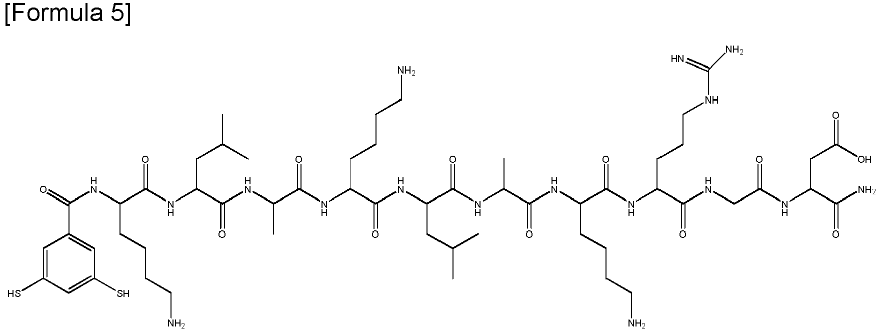

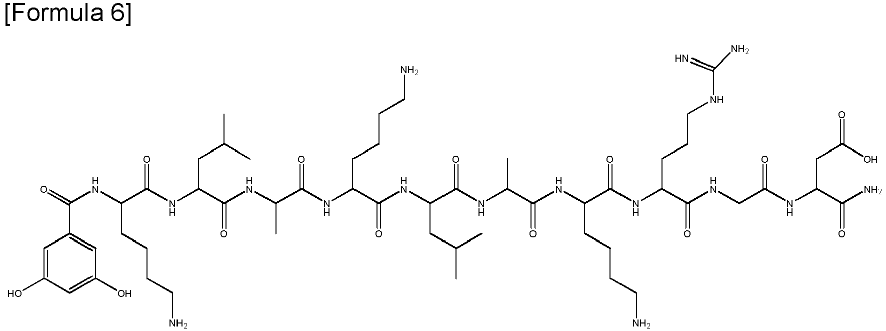

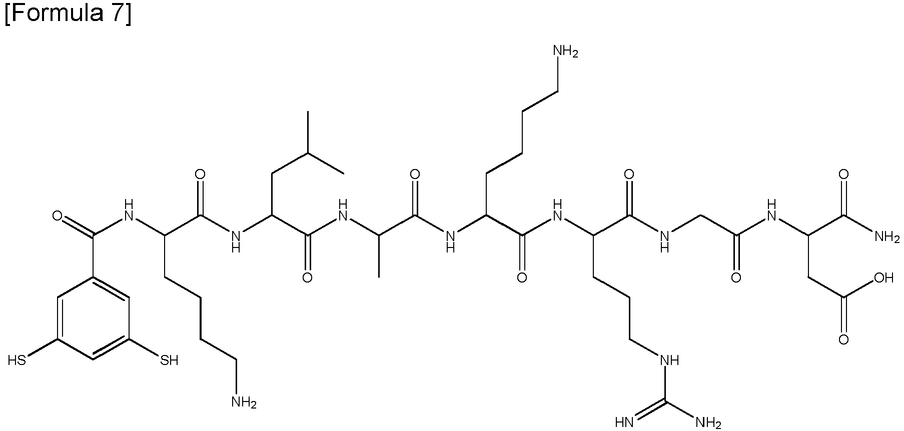

8. The compound or pharmaceutically acceptable salt thereof of claim 1, wherein R₁ of Formula 1 or R₄ of Formula 2 is oxidized to form a bond with a plurality of other compounds.

9. The compound or pharmaceutically acceptable salt thereof of claim 8, wherein the oxidation is caused by reactive oxygen species (ROS).

10. A pharmaceutical composition for preventing or treating aging-related diseases, the pharmaceutical composition comprising, as an active ingredient, the compound or pharmaceutically acceptable salt thereof of claim 1.

11. The pharmaceutical composition of claim 10, wherein the aging-related disease is at least one selected from senile cardiovascular disease and disorder, senile lung disease, idiopathic pulmonary fibrosis, chronic obstructive pulmonary disease, osteoarthritis, senile eye disease, and skin aging.

12. The pharmaceutical composition of claim 11, wherein the senile eye disease is senile macular degeneration.

13. The pharmaceutical composition of claim 10, wherein the compound of the pharmaceutical composition induces apoptosis in mitochondria in senescent cells.

14. A method of preventing or treating aging-related diseases, comprising administering, to a subject in need thereof, the compound or pharmaceutically acceptable salt thereof of claim 1.

15. A use of the compound or pharmaceutically acceptable salt thereof of claim 1 in the preparation of a composition for preventing or treating aging-related diseases.
